(19) Europäisches Patentamt
European Patent Office
Office européen des brevets

(11) Veröffentlichungsnummer: **0 005 757**
**B1**

(12) **EUROPÄISCHE PATENTSCHRIFT**

(21) Anmeldenummer: **79101466.5**

(22) Anmeldetag: **14.05.79**

(51) Int. Cl.³: **C 07 C 49/76,**
C 07 C 49/80, C 09 B 3/04

(54) **Verfahren zur Herstellung von Benzanthronen**

(30) Priorität: **26.05.78 DE 2823160**

(43) Veröffentlichungstag der Anmeldung:
**12.12.79 Patentblatt 79/25**

(45) Bekanntmachung des Hinweises auf die Patenterteilung:
**15.10.80 Patentblatt 80/21**

(84) Benannte Vertragsstaaten:
**CH DE FR GB IT**

(56) Entgegenhaltungen:
**CH - A - 178 942**
**FR - A - 2 371 413**
**GB - A - 297 129**
**US - A - 1 749 519**
**US - A - 1 934 221**

(73) Patentinhaber: **BAYER AG**
**Zentralbereich Patente, Marken und Lizenzen**
**D - 5090 Leverkusen 1, Bayerwerk (DE)**

(72) Erfinder: **Schroeder, Josef, Dr.**
**Paul-Klee-Strasse 64**
**D - 5090 Leverkusen 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

# Verfahren zur Herstellung von Benzanthronen

Die Erfindung betrifft ein Verfahren zur Herstellung von Benzanthronen.

Es ist bekannt, daß Benzanthron in einer gemeinsamen Reaktion aus Anthrachinon, Reduktionsmittel und Glycerin entsteht. [N. N. Woroskzow, Grundlagen der Synthese von Zwischenprodukten und Farbstoffen, S. 907 ff., Akademie-Verlag, Berlin 1966]. Als Reduktionsmittel werden in der Literatur Metalle wie Eisen, Aluminium oder Kupfer empfohlen.

Es wurde nun gefunden, daß man Benzanthrone der Formel

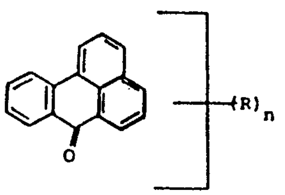

(I)

in der

R Alkyl und Halogen bedeutet und

n für 0, 1, 2 oder 3 steht,

erhält, wenn man Anthrachinone der Formel

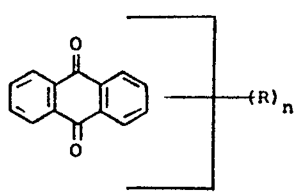

(II)

in der

R und n die oben angegebenene Bedeutung haben, mit Acrolein oder Verbindungen, aus denen unter den Reaktionsbedingungen Acrolein entstehen kann, in Gegenwart von Phosphor oder reduzierend wirkenden Phosphorverbindungen in Schwefelsäure höherer Konzentration bei erhöhter Temperatur umsetzt, und den Ansatz in an sich bekannter Weise auf die Benzanthrone der Formel (I) hin aufarbeitet.

Die als Ausgangsprodukte eingesetzten Anthrachinone der Formel (II) sind bekannt [siehe z.B. Elsevier's Encyclopaedia of Organic Chemistry, Band 13, S. 400 ff.] oder analog zu literaturbekannten Verfahren herstellbar.

Das Verfahren eignet sich in besondere Weise zur Herstellung von Benzanthronen der Formel I, bei denen R $C_1$—$C_4$-Alkyl wie Methyl, Äthyl, n-Propyl, i-Propyl, n-Butyl, sec.-Butyl, tert.-Butyl und Halogen wie Chlor und Brom bezeichnet.

n steht bevorzugt für 0, 1 oder 2.

Besonders gut eignet sich das neue Verfahren zur Herstellung von Benzanthron selbst oder von Verbindungen der Formel

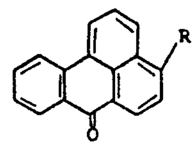

(III)

in der R die oben angegebene Bedeutung besitzt.

Das zur Ringangliederung verwendete Acrolein kann als solches in die Reaktion eingesetzt werden. Vorzugsweise verwendet mann zur Acroleinerzeugung jedoch Verbindungen, aus denen in Schwefelsäure bei erhöhter Temperatur Acrolein entsteht, wozu sich insbesondere Glycerin, aber auch Glycerinester wie Glycerinmono-, di- und -triacetat eignen.

Als Reduktionsmittel werden Phosphorverbindungen niedriger Oxidationsstufen, bevorzugt solche der Oxidationsstufe + 1 vorzugsweise in Form der Alkalisalze der unterphosphorigen Säure eingesetzt. Die Reduktion gelingt auch mit phosphoriger Säure, sowie deren Salzen und Estern, z.B. Trimethyl- oder Triäthylphosphit. Auch elementarer Phosphor eignet sich zur Reduktion.

Während man beim Einsatz von Natriumhypophosphit 2—4 Reduktionsäquivalente pro Mol Anthrachinon einsetzt, bevorzugt 2,5—3,5, d.h. 0,5 bis 1 Mol Natriumhypophosphit pro Mol Anthrachinon, benötigt man bei Verwendung von phosphoriger Säure, deren Ester oder elementarem Phosphor mehr als den molaren Verhältnissen entspricht. Die Mengen an einzusetzendem reduktionsmittel lassen sich für den Fachmann unschwer ermitteln.

Pro Gewichtsteil Anthrachinonverbindung enthält der Reaktionsansatz vorzugsweise 2 bis 10 Gewichtsteile, insbesondere 3 bis 5 Gewichtsteile, Schwefelsäure, die etwa 80 bis 100%ig sein sollte.

Man arbeitet vorzugsweise in einem Temperaturbereich von 80 bis 140°C, besonders bevorzugt bei 100 bis 120°C. Die Reaktionsdauer beträgt vorzugsweise 1 bis 10 h; die günstigsten Umsetzungszeiten liegen bei 2 bis 4 h.

Das Verfahren kann beispielsweise wie folgt durchgeführt werden:

Die Anthrachinonverbindung der Formel (II) wird in Schwefelsäure höherer Konzentration, z.B. 96% oder Monohydrat, bei erhöhter Temperatur (etwa 80—110°C) gelöst.

a) Man fügt nun zu dieser Lösung zunächst das Reduktionsmittel, z.B. Phosphor, phosphorige Säure und deren Salze oder Ester, Salze der unterphosphorigen Säure und fügt anschließend Acrolein entstehen kann, wie z.B. Glycerin oder Glycerinester, zu.

b) Eine vorteilhaftere Verfahrensweise besteht darin, geeignete Phosphorverbindungen,

wie z.B. phosphorige Säure oder Natriumhypophosphit in Glycerin zu lösen und diese Lösung je nach Fortgang der Reaktion zu dosieren, wobei sich besonders Vorteile in der Temperaturführung ergeben.

Durch Eingießen der Reaktionsschmelzen in Wasser werden die rohen Benzanthrone gewonnen. Nach Auskochung mit schwachem Alkali resultiert ein Produkt von hoher Reinheit in Ausbeuten >90% d.Th., bei niedrigen Gehalten ist Beimengung z.B. nicht umgesetztes Anthrachinon.

Die Umwandlung der Antrachinone in Benzanthrone erfolgt nahezu ohne Nebenreaktionen wie aus Dünnschichtchromatogrammen zu ersehen ist.

Das neue Verfahren ist einfach in der Durchführung, die Reaktion wird durch das Arbeiten in homogener Lösung leicht kontrollierbar. Wesentliche Vorteile gegenüber bekannten Verfahren bestehen auch in der einfachen Aufarbeitung und Isolation der Reaktionsprodukte.

Die großen Mengen Wasserstoff, die beim Arbeiten mit metallischen Reduktionsmitteln anfallen, treten nicht auf.

Die Menge der anfallenden Dünnsäure ist wegen des wesentlich konzentrierten Ansatzes geringer; die ökologische Belastung der anfallenden Abwässer ist deutlich geringer.

Erwähnenswert ist weiterhin die hohe Qualität der nach dem erfindungsgemäßen Verfahren herstellbaren Benzanthrone, die bei herkömmlichen Verfahren nur durch zusätzliche und aufwendige Reinigungsschnitte wie etwa Vakuumsublimation erzielt werden kann.

Die nach dem erfindungsgemäßen Verfahren herstellbaren Benzanthrone sind bekannt [siehe z.B. Elsevier's Encyclopaedia of Organic Chemistry, Band 14, S. 362 ff.] und finden Verwendung als Zwischenprodukte für wertvolle Küpenfarbstoffe.

Beispiel 1

In 1000 g konz. Schwefelsäure werden bei 80—110° 208 g Anthrachinon gelöst. In diese Lösung werden 96 g Natriumhypophosphit, gelöst in 240 g Glycerin so zugegeben, daß 100°C gehalten werden können. Man heizt dann auf 120°C und hält 3 Stunden bei dieser Temperatur. Anschließend gießt man die Schmelze in 3 l Wasser und saugt das abgeschiedene Produkt ab. Man wäscht mit heißem Wasser bis zum nahezu farblosen Ablauf und suspendiert den Filterkuchen dann in 1,5 l Wasser, stellt schwach alkalisch und kocht auf. Nach erneuter Filtration. Wäsche mit heißem Wasser und Trocknung bei 100°C resultieren 200—225 g Benzanthron 92—97%ig ≈80—93% d.Th.

Beispiel 2

Analog zu Beispiel 1 lassen sich glatt umsetzen: 2-Methylanthrachinon zu einem Gemisch von Monomethyl-benzanthronen (zu einem großen Teil 4-Methylbenzanthron); 2-Äthylanthrachinon zu einem Gemisch von Monoäthylbenzanthronen (zu einem großen Teil 4-Äthylbenzanthron); 1,5-Dichloranthrachinon zu 6,10-Dichlorbenzanthron; 2-Chloranthrachinon zu einem Gemisch von Monochlorbenzanthronen.

Aus den Gemischen lassen sich die Einzelverbindungen durch fraktionierte Kristallisation gewinnen.

**Patentansprüche**

1. Verfahren zur Herstellung von Benzanthronen der Formel

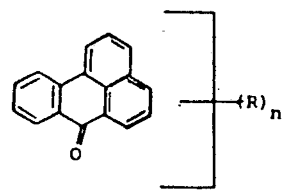

(I)

in der
R Alkyl und Halogen bezeichnet und
n für 0, 1, 2 oder 3 steht,
dadurch gekennzeichnet, daß man Anthrachinone der Formel

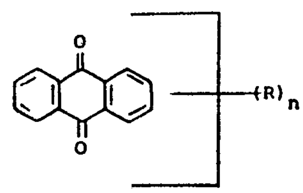

(II)

in der R und n die oben angegebene Bedeutung besitzen, mit Acrolein oder Verbindung, aus denen unter den Reaktionsbedingungen Acrolein entstehen kann, in Gegenwart von Phosphor oder reduzierend wirkenden Phosphorverbindungen in Schwefelsäure höherer Konzentration bei erhöhter Temperatur umsetzt und den Reaktionsansatz in an sich bekannter Weise auf die Benzanthrone der Formel (I) hin aufarbeitet.

2. Verfahren gemäß Anspruch 1, dadurch gekennzeichnet, daß man pro Mol Anthrachinon der Formel (II) 2 bis 4 Reduktionsäquivalente Phosphor oder Phosphorverbindung und pro Gewichtsteil Anthrachinon der Formel (II) 2 bis 10 Gewichtsteile 80 bis 100%ige Schwefelsäure einsetzt und die Reaktion im Temperaturbereich von 80 bis 140°C durchführt.

3. Verfahren gemäß den Ansprüchen 1 und 2, dadurch gekennzeichnet, daß man Glycerin oder ein Glycerinacetat verwendet.

4. Verfahren gemäß den Ansprüchen 1 bis 3, dadurch gekennzeichnet, daß man Natriumhypophosphit verwendet.

5. Verfahren gemäß den Anspruchen 1 bis 4, dadurch gekennzeichnet, daß man die reduzierende Phosphorverbindung in Glycerin löst und die Lösung zum Reaktionsansatz aus Antrachinon der Formel (II) und Schwefelsäure gibt.

## Claims

1. Process for the preparation of benzanthrones of the formula

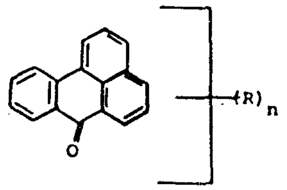

(I)

in which

R designates alkyl or halogen and

n represents 0, 1, 2, or 3,

characterised in that anthraquinones of the formula

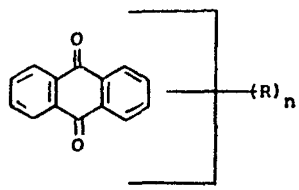

(II)

in which

R and n have the meaning indicated above, are reacted with acrolein or compounds from which acrolein can be formed under the reaction conditions, in the presence of phosphorus or phosphorus compounds having a reducing action, in relatively highly concentrated sulphuric acid at elevated temperature, and the reaction mixture is worked up in the manner which is in itself known to give the benzanthrones of the formula (I).

2. Process according to Claim 1, characterised in that 2 to 4 reduction equivalents of phosphorus or phosphorus compound are employed per mol of anthraquinone of the formula (II), 2 to 10 parts by weight of 80 to 100% strength sulphuric acid are employed per part by weight of anthraquinone of the formula (II), and the reaction is carried out in the temperature range from 80 to 140°C.

3. Process according to Claims 1 and 2, characterised in that glycerol or a glycerol acetate is used.

4. Process according to Claims 1 to 3, characterised in that sodium hypophosphite is used.

5. Process according to Claims 1 to 4, characterised in that the reducing phosphorus compound is dissolved in glycerol and the solution is added to the reaction mixture of anthraquinone of the formula (II) and sulphuric acid.

## Revendications

1. Procédé de production de benzanthrones de formule:

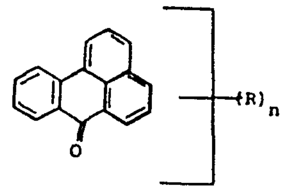

(I)

dans laquelle

R représente un groupe alkyle et un halogène et

*n* a la valeur 0, 1, 2 ou 3,

caractérisé en ce qu'on fait réagir à température élevée des anthraquinones de formule:

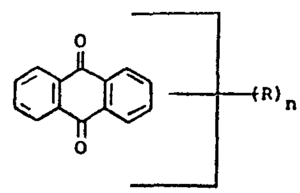

(II)

dans laquelle R et *n* ont la définition indiquée ci-dessus, avec l'acroléine ou des composés à partir desquels de l'acroléine peut être formée dans les conditions réactionnelles, en présence de phosphore ou de composés phosphorés à action réductrice, dans de l'acide sulfurique à concentration élevée et on traite le mélange réactionnel d'une manière connue pour obtenir des benzanthrones de formule (I).

2. Procédé suivant la revendication 1, caractérisé en ce qu'on utilise, par mole d'anthraquinone de formule (II), 2 à 4 équivalents de réduction de phosphore ou de composé phosphoré et, par partie en poids d'anthraquinone de formule (II), 2 à 10 parties en poids d'acide sulfurique à 80—100% et on conduit la réaction dans la plage de température de 80 à 140°C.

3. Procédé suivant l'une des revendications 1 et 2, caractérisé en ce qu'on utilise du glycérol ou un acétate de glycérol.

4. Procédé suivant l'une quelconque des revendications 1 à 3, caractérisé en ce qu'on utilise de l'hypophosphite de sodium.

5. Procédé suivant l'une quelconque des revendications 1 à 4, caractérisé en ce qu'on dissout le composé phosphoré réducteur dans du glycérol et on ajoute la solution au mélange réactionnel formé d'anthraquinone de formule (II) et d'acide sulfurique.